# EUROPEAN PATENT SPECIFICATION

(11) **EP 3 950 949 B1**
(45) Date of publication and mention of the grant of the patent: **04.12.2024**
(21) Application number: 20776454.9
(22) Date of filing: 26.03.2020
(51) Int. Cl.: C12N 15/877, A01K 67/027, C12N 5/0789, C12N 15/24, C12N 15/27

(54) **IMMUNODEFICIENT RODENT**
IMMUNDEFIZIENTE NAGER
RONGEUR IMMUNODÉFICIENT

(30) Priority: 28.03.2019 JP 2019062919
(43) Date of publication of application: 09.02.2022
(73) Proprietor: Central Institute for Experimental Medicine and Life Science, Kawasaki-shi, Kanagawa 210-0821 (JP)
(72) Inventor: ITO, Ryoji, Kawasaki-shi, Kanagawa 210-0821 (JP)
(74) Representative: D Young & Co LLP
(86) International application number: PCT/JP2020/013635
(87) International publication number: WO 2020/196742

(56) References cited:
- WO-A1-2020/067199
- ITO ET AL: "PS2-1-2, Development of human neutrophils in hG-CSF knockin NOG mouse transferred with human HSC", RINSHO KETSUEKI - JAPANESE JOURNAL OF CLINICAL HEMATOLOGY; PROGRAMS AND ABSTRACTS OF THE 80TH ANNUAL MEETING OF THE JAPANESE SOCIETY OF HEMATOLOGY; OCTOBER 12-14, 2018, NIHON RINSHO KETSUEKI GAKKAI, JP, vol. 59, no. 9, 13 September 2018 (2018-09-13), XP009531516, ISSN: 0485-1439
- R. ITO ET AL: "Establishment of a Human Allergy Model Using Human IL-3/GM-CSF-Transgenic NOG Mice", THE JOURNAL OF IMMUNOLOGY, vol. 191, no. 6, 15 September 2013 (2013-09-15), US, pages 2890 - 2899, XP055508668, ISSN: 0022-1767, DOI: 10.4049/jimmunol.1203543
- ITO RYOJI ET AL: "A humanized mouse model to study asthmatic airway inflammation via the human IL-33/IL-13 axis", JCI INSIGHT, vol. 3, no. 21, 2 November 2018 (2018-11-02), pages 1 - 15, XP055777010, Retrieved from the Internet <URL:https://www.ncbi.nlm.nih.gov/pmc/articles/PMC6238753/pdf/jciinsight-3-121580.pdf> DOI: 10.1172/jci.insight.121580
- R. ITO ET AL: "Human G-CSF knockin NOG mice support the differentiation of human neutrophils and recapitulate an emergency granulopoiesis after bacterial infection | The Journal of Immunology", 1 May 2019 (2019-05-01), XP055979472, Retrieved from the Internet <URL:https://www.jimmunol.org/content/202/1_Supplement/131.31> [retrieved on 20221109]
- ANTHONY RONGVAUX ET AL: "Development and function of human innate immune cells in a humanized mouse model", NATURE BIOTECHNOLOGY, 16 March 2014 (2014-03-16), XP055112690, ISSN: 1087-0156, DOI: 10.1038/nbt.2858
- YOSHIHIRO INOUE ET AL: "Activating Fcgamma Receptors Participate in the Development of Autoimmune Diabetes in NOD Mice", THE JOURNAL OF IMMUNOLOGY, vol. 179, no. 2, 15 July 2007 (2007-07-15), US, pages 764 - 774, XP055743305, ISSN: 0022-1767, DOI: 10.4049/jimmunol.179.2.764
- ITO, R. : "PS2-1-2, Development of human neutrophils in hG-CSF knockin NOG mouse transferred with human HSC", JAPANESE JOURNAL OF CLINICAL HEMATOLOGY, vol. 59, no. 9, 13 September 2018 (2018-09-13), JP, pages 560 (1694), XP009531516, ISSN: 0485-1439
- ITO, R. ET AL.: "Establishment of a Human Allergy Model Using Human IL -3/GM- CSF-Transgenic NOG Mice", J IMMUNOL, vol. 191, 16 August 2013 (2013-08-16), pages 2890 - 2899, XP055508668, DOI: 10.4049/jimmunol.1203543
- ITO RYOJI; MARUOKA SHUICHIRO; SODA KAORI; KATANO IKUMI; KAWAI KENJI; YAGOTO MIKA; HANAZAWA ASAMI; TAKAHASHI TAKESHI; OGURA TOMOYUK: "A humanized mouse model to study asthmatic airway inflammation via the human IL -33/ IL -13 axis", JCI INSIGHT, vol. 3, no. 21, 2 November 2018 (2018-11-02), pages 1 - 15, XP055777010, DOI: 10.1172/jci.insight.121580
- INOUE, Y. ET AL.: "Activating Fc gamma Receptors Participate in the Development of Autoimmune Diabetes in NOD Mice", J IMMUNOL, vol. 179, 2007, pages 764 - 774, XP055743305
- ANONYMOUS: "Business year (61st period)", BUSINESS REPORT, vol. 61, JP, pages 1 - 65, XP009531517, Retrieved from the Internet <URL:https://www.ciea.or.jp/about/pdf/report/61_report.pdf> [retrieved on 20200601]
- ITO, R. : "PS2-1-2, Development of human neutrophils in hG-CSF knockin NOG mouse transferred with human HSC", JAPANESE JOURNAL OF CLINICAL HEMATOLOGY, vol. 59, no. 9, 13 September 2018 (2018-09-13), pages 134 (1268), XP009531515, ISSN: 0485-1439
- ITO, R. : "PS2-1-2, Development of human neutrophils in hG-CSF knockin NOG mouse transferred with human HSC", RINSHO KETSUEKI - JAPANESE JOURNAL OF CLINICAL HEMATOLOGY, NIHON RINSHō KETSUEKI GAKKAI, JP, vol. 59, no. 9, 13 September 2018 (2018-09-13) - 14 December 2018 (2018-12-14), JP , pages 134 (1268), XP009531515, ISSN: 0485-1439

## Description

### Technical Field

The present invention relates to a rodent having a human G-CSF gene knock-in at a G-CSF receptor locus thereof, which is an immunodeficient rodent deficient in G-CSF receptor function by knock-in of the human G-CSF gene at the G-CSF receptor locus thereof, wherein the human G-CSF is expressed and the rodent G-CSF receptor is not expressed, and further deficient in Fcer1g and Fcgr2b, which are constituent molecules of a receptor that binds to an Fc region of an antibody, obtained by further deleting the Fcer1g and Fcgr2b in the rodent.

### Background Art

As an extremely excellent immunodeficient mouse, a NOG mouse is known (Patent Literature 1). Humanized NOG mice are prepared by transplanting human cells or tissues into NOG mice. In order to reproduce the human innate immune system, it is necessary to develop a mouse, in which human granulocytes such as human neutrophils can circulate in the periphery for a long time. For the purpose, it is known that human hematopoietic stem cells are transplanted and human G-CSF protein is administered to prepare mice (Non Patent Literatures 1, 2); however, human neutrophils engrafted but transient and mouse neutrophils increases. A system dedicated to the human innate immune system cannot be obtained. This is a matter of great concern.

Eosinophils and basophils are the cells of the innate immune system involved in allergic diseases. There are mice in which human eosinophils and human basophils differentiate (Non Patent Literatures 3, 4). However, in any mouse, human eosinophils and human basophils differentiate only in small amounts. Rodents, in which sufficient amounts of human eosinophils and basophils are maintained for a long term, have not yet been obtained.

### Citation List

### Patent Literature

Patent Literatures 1: International Publication No. WO2002/043477

### Non Patent Literature

Non Patent Literature 1: Tanaka S. et al., J Immunol. 2012 Jun 15; 188(12): 6145-55
Non Patent Literature 2: Coughlan AM et al., Stem Cells Dev. 2016 Apr 1; 25(7): 530-41
Non Patent Literature 3: Ito R et al., Journal of immunology 2013; 191: 2890-2899.
Non Patent Literature 4: Ito R et al., JCI Insight. 2018 Nov 2; 3(21)

Further documents identified during the Search and Examination procedure are outlined below.

Ito et al., Rinsho Ketsueki - Japanese Journal of clinical hematology; Programs and abstracts of the 80th annual meeting of the Japanese society of hematology; October 12-14, 2018, Nihon Rinsho Ketsueki Gakkai, JP. 2018. Ito et al. is a conference booklet from the Japanese Society of Hematology, disclosing abstracts of the posters to be presented. The abstract forPS2-1-2 discloses a novel NOG mouse strain with human G-CSF knock-in targeting the G-CSF receptor locus to induce mature human neutrophils in their circulation.

Rongvaux et al., Nature Biotechnology. 2014 March 16. Rongvaux et al. discloses development of humanized mice with human innate immune cells.

Inoue et al., The Journal of Immunology. 2007; 179(2): 764-774. Inoue discloses the generation of various FcyR-deficientNOD lines.

### Summary

### Technical Problem

An object of the present invention is to provide a humanized mouse in which the human innate immune system can be reproduced and maintained for a long term without activating the mouse innate immune system.

### Solution to Problem

The present inventors knocked-in a human G-CSF cytokine known as a neutrophil-inducing factor in an immunodeficient mouse in order to promote engraftment of human neutrophils. At this time, to prevent a cross reaction between a human G-CSF and a mouse G-CSF receptor to activate the mouse innate immune system, a human G-CSF gene was knocked-in at a mouse G-CSF receptor locus to provide deficiency of the mouse G-CSF receptor.

As a result, the inventors have successfully produced a humanized mouse in which a human G-CSF is present in serum and the human hematopoietic system is reconstructed in an immunodeficient mouse.

The inventors further deleted a receptor that binds to the Fc region of an antibody in the humanized mouse in which a human G-CSF is present in serum and the human hematopoietic system is reconstructed in an immunodeficient mouse, to produce a humanized mouse in which human neutrophils much more differentiate than in the aforementioned mouse.

Furthermore, the inventors deleted a receptor that binds to the Fc region of an antibody in a hIL-3/hGM-CSF Tg mouse and a hIL-3/hGM-CSF/hIL-5 Tg mouse in which small amounts of human eosinophils and basophils differentiate to produce a humanized mouse in which the eosinophils and basophils much more differentiate.

The present invention is set out in the appended set of claims.

In a first aspect there is provided a rodent having a human G-CSF gene knock-in at a G-CSF receptor locus thereof, as defined in claim 1.

In a second aspect, there is provided a rodent model for a human pathogen infectious disease, obtained by infecting the humanized rodent with a bacterium or virus, as set out in claim 7.

### Advantageous Effects

The rodent described herein, which is a knock-in rodent prepared by knocking in a human G-CSF gene at a rodent's G-CSF receptor locus and transplanting human hematopoietic stem cells thereinto, is a humanized rodent in which the human immune system is reproduced and human neutrophils and monocytes circulate in the periphery. Such a rodent can be used in studies for human immune system. The mouse described herein is the first humanized mouse in which human neutrophils circulate in the peripheral blood for a long term and useful in studies for a congenital defense mechanism against human bacterial infection. The rodent further infected with a pathogen can be used as a rodent model for a human infectious disease.

A humanized mouse, in which human eosinophils and basophils are highly differentiated and maintained for a long term, can be used for reproducing a severe human allergic disease such as refractory asthma that has been difficult to treat.

### Brief Description of Drawings

[Figure 1] Figure 1 shows the structure of a human G-CSF knock-in targeting vector.
[Figure 2] Figure 2 shows the mouse G-CSF receptor genotype of the human G-CSF knock-in mouse.
[Figure 3] Figure 3 shows human G-CSF concentrations (average value) in serum of human G-CSF knock-in mice.
[Figure 4] Figure 4 shows loss of expression of the mouse G-CSF receptor on granulocytes of human G-CSF knock-in mice.
[Figure 5] Figure 5 shows mouse neutrophil counts in the blood of human G-CSF knock-in mice.
[Figure 6] Figure 6 shows the analysis results of radiation sensitivity of human G-CSF knock-in mice.
[Figure 7] Figure 7 shows the ratios of human cells in the blood of human G-CSF knock-in mice 12 weeks after humanization with human hematopoietic stem cells.
[Figure 8] Figure 8 shows the ratio and count of human CD45+ cells in the blood of human G-CSF knock-in mice 4, 8 and 12 weeks after humanization with human hematopoietic stem cells.
[Figure 9] Figure 9 shows the ratio and count of human neutrophils in the blood of human G-CSF knock-in mice 4, 8 and 12 weeks after humanization with human hematopoietic stem cells.
[Figure 10] Figure 10 shows the ratio and count of human monocytes in the blood of human G-CSF knock-in mice 4, 8 and 12 weeks after humanization with human hematopoietic stem cells.
[Figure 11] Figure 11 shows morphology of human neutrophils of a human G-CSF knock-in mouse humanized with human hematopoietic stem cells analyzed by Giemsa staining.
[Figure 12] Figure 12 shows the analysis results of reactive oxygen species (ROS) production ability of human neutrophils of human G-CSF knock-in mice humanized with human hematopoietic stem cells.
[Figure 13] Figure 13 shows productions of human cytokines in serum of human G-CSF knock-in mice humanized with human hematopoietic stem cells.
[Figure 14] Figure 14 shows the ratio of human neutrophils 4h after administration of zymosan to human G-CSF knock-in mice humanized with human hematopoietic stem cells.
[Figure 15] Figure 15 shows the ratios of individual human cells 4h after administration of zymosan to human G-CSF knock-in mice humanized with human hematopoietic stem cells.
[Figure 16] Figure 16 shows measurement results of human cytokine levels in serum 4h after administration of zymosan to human G-CSF knock-in mice humanized with human hematopoietic stem cells.
[Figure 17] Figure 17 shows the ratios of individual human cells 4h after administration of *Escherichia coli* to human G-CSF knock-in mice humanized with human hematopoietic stem cells.
[Figure 18-1] Figure 18-1 shows measurement results of mouse cytokine levels in serum 4h after administration of *Escherichia coli* to human G-CSF knock-in mice humanized with human hematopoietic stem cells.
[Figure 18-2] Figure 18-2 shows measurement results of human cytokine levels in serum 4h after administration of *Escherichia coli* to human G-CSF knock-in mice humanized with human hematopoietic stem cells.
[Figure 19] Figure 19 shows the ratio of human neutrophils in human leukocytes of peripheral blood of hG-CSF KI, FcR KO mice.
[Figure 20] Figure 20 shows the ratio of human eosinophils in human leukocytes of peripheral blood of hIL-3/hGM-CSF Tg, FcRKO mice.
[Figure 21] Figure 21 shows the ratio of human eosinophils in human leukocytes of peripheral blood of hIL-3/hGM-CSF/hIL-5 Tg, FcR KO mice.
[Figure 22] Figure 22 shows the ratio of human basophils in human leukocytes of peripheral blood of hIL-3/hGM-CSF/hIL-5 Tg, FcR KO mice.
[Figure 23] Figure 23 shows the relationship between mouse strains and the ratios of human granulocytes in human leukocytes.

### Description

The following passages are provided for illustration purposes only.

1. Preparation of a knock-in rodent having a human G-CSF gene knock-in at a rodent's G-CSF receptor locus and a humanized rodent in which human neutrophils derived from human hematopoietic stem cells (HSC) transplanted circulate in the periphery

Described herein is a knock-in rodent having a human G-CSF (granulocyte-colony stimulating factor) gene knock-in. In the knock-in rodent described herein, a human G-CSF gene is knocked-in at a G-CSF receptor locus that a rodent originally has, and thus the G-CSF receptor that the rodent originally has is not expressed, or even if it is expressed, the receptor is deficient in the function. The knock-in rodent as described herein can be prepared in one step by knocking in the human G-CSF gene, while defunctionalizing the G-CSF receptor of a rodent.

The DNA sequence of the human G-CSF gene is represented by SEQ ID NO: 1 and the DNA sequence of the mouse G-CSF receptor gene is represented by SEQ ID NO: 2.

The knock-in (gene knock-in) is a genetic engineering technique for inserting a DNA sequence encoding a protein at a predetermined locus on a chromosome of an organism.

Human G-CSF can bind to a G-CSF receptor of a rodent. Accordingly, when a G-CSF receptor is expressed in a rodent having a human G-CSF gene knock-in, if the human G-CSF gene knock-in in a rodent is expressed within the body of the rodent, human G-CSF binds to the G-CSF receptor expressed in, e.g., neutrophil progenitor cells of the rodent, with the result that the neutrophils of the rodent may proliferate. However, the G-CSF receptor of the knock-in rodent as described herein is already defunctionalized, the human G-CSF expressed in a knock-in rodent will not bind to the rodent's G-CSF receptor.

In the blood of the knock-in rodent having a human G-CSF gene at a rodent's G-CSF receptor locus, the human G-CSF is present. If human hematopoietic stem cells (HSC) are transplanted into the knock-in rodent, the human G-CSF stimulates human hematopoietic stem cells within the body of the rodent, with the result that human neutrophils and monocytes differentiate, proliferate and circulate in the peripheral blood. More specifically, when human HSCs are transplanted into a rodent having a human G-CSF gene knock-in at a rodent's G-CSF receptor locus, the human hematopoietic system is reconstructed within the body of the rodent. In this sense, the rodent is a humanized rodent in which the human hematopoietic system is reconstructed. In the rodent, human neutrophils and monocytes circulate in the periphery.

Disclosed herein is a method for preparing a knock-in rodent having a human G-CSF gene, comprising knocking in a human G-CSF gene at a G-CSF receptor locus of a rodent.

Disclosed herein is a method for preparing a knock-in rodent having a human G-CSF gene at a G-CSF receptor locus thereof, wherein human HSCs are transplanted, and human neutrophils are differentiating and circulating in the periphery, by transplanting human HSCs into a knock-in rodent having a human G-CSF gene.

Described herein is a knock-in rodent having a human G-CSF gene at a rodent's G-CSF receptor locus, wherein human HSCs are transplanted, and human neutrophils are differentiating and circulating in the periphery.

As described herein, examples of the rodent include, but are not limited to, a mouse, a rat, a guinea pig, a hamster, a rabbit and a nutria. Of them, a mouse is preferable.

The nucleotide sequence of the human G-CSF gene is represented by SEQ ID NO: 1 and the nucleotide sequence of the mouse G-CSF receptor gene is represented by SEQ ID NO: 2.

A humanized rodent, as described herein.in which the human neutrophils circulate in the periphery is a rodent that does not eliminate human cells such as human neutrophils by the immune system, and more specifically, a rodent in which the immune response to humans is inactivated. As such a rodent, a rodent having a lowered or deficient immune function and inactivated in immune response to humans is mentioned. For example, an immunodeficient rodent, can be used.

The immunodeficient rodent is a rodent having lowered or deficient immune function, and more specifically, a rodent which lacks a part or whole of T cells, B cells, NK cells, dendritic cells and macrophages. The immunodeficient rodent can be prepared by irradiating the whole body with X-rays. Alternatively, a rodent genetically deficient in immune function can be used.

Examples of the immunodeficient mouse that can be used include a nude mouse, a NOD/SCID mouse, a Rag2 knockout mouse, a SCID mouse administered with an asialo-GM1 antibody or TMβ1, and a mouse irradiated with X-rays. Also, knockout mice (hereinafter referred to as dKO (double knockout) mice) obtained by crossing a NOD/SCID mouse or a Rag2 knockout mouse with an IL-2Ry knockout mouse, can be used. For example, a dKO mouse (Rag2 KO, IL-2R^{null}) can be used. The dKO mouse having a genetic background of Balb/c is referred to as a Balb/c dKO mouse; a dKO mouse having a genetic background of NOD is referred to as a NOD dKO mouse. The genetic background of a mouse is not limited to these. Not only C57BL/6, C3H, DBA2 and IQI strains but also a strain having a SCID mutation and an IL-2Ry knockout, or a Rag2 knockout and an IL-2R γ knockout mutation, as well as a deficiency of Jak3 protein responsible for signal transduction downstream of common γ chain of an IL-2 receptor, have the similar phenotype to IL2Rγ^{null}. Because of this, a knockout mouse obtained by crossing a Rag2 knockout mouse with a Jak3 knockout; a knockout mouse obtained by crossing a SCID mutation and a Jak3 knockout; and an inbred mouse, a non-inbred mouse and a crossbreeding (F1 hybrid) mouse obtained by crossing them, may be used.

In order to exclude an effect by immune cells such as NK cells observed in the mouse, a SCID mouse administered with an asialo GM1 antibody is used as mentioned above. Other than this embodiment, there is another mouse to be used described herein. This is a genetically modified immunodeficient mouse lacking the IL-2 receptor γ chain due to introduction of a mutation in an IL-2 receptor γ chain gene and having a SCID mutation of a gene responsible for reconstruction of antigen receptor genes of a T cell and a B cell at both allele loci. Examples of such a mouse include mice such as a NOD mouse derived from a NOD/SCID mouse and having a knock-out of common γ chain of IL-2 receptor (NOD/SCID/γc^{null} (NOD/Shi-scid, IL-2Rγ KO mouse)), a NSG mouse (NOD/Scid/IL2Rγ^{null} (NOD.Cg-Prkdc^{scid}IL2rg^{tm1Wjl}/SzJ)) and a NCG mouse (NOD-Prkdc^{em26Cd52}IL2rg^{em26Cd22}/NjuCrl). Further, a genetically modified immunodeficient mouse NOJ mouse (NOD/Scid/Jak3^{null} (NOD.Cg-Prkdc^{scid}Jak3^{tm1card})) deficient in Jak3 due to introduction of a mutation in a Jak3 gene and having a SCID mutation of a gene responsible for reconstruction of an antigen receptor gene of a T cell and a B cell in both allele loci, can be used. Hereinafter, animals (mice) deficient in function of a Prkdc gene and a gene product thereof due to e.g., a scid mutation; and losing a normal function of an IL2Rγ gene product due to deletion or mutation of an IL2Rγ gene or loss-of-function of a gene present downstream of signal transduction and a gene product thereof will be referred to as NOG mice ("NOG mouse" is a registered trademark) and can be used as a host. Since lymphocytes are not present in these mice, the NOG mice have neither the NK activity nor dendritic cell function. A method for producing NOG mice is described in WO2002/043477. A method for producing NSG mice is described in Ishikawa F. et al., Blood 106: 1565-1573, 2005. A method for producing NCG mice is described in Zhou J. et al. Int J Biochem Cell Biol 46: 49-55, 2014. A method for producing the NOJ mice is described in Okada S. et al., Int J Hematol 88: 476-482, 2008.

A method for knocking in the human G-CSF gene is not limited, and for example, a gene knock-in can be made by homologous recombination and genome editing. The following example disclosing the preparation of the humanized rodent described herein is present for illustration purposes only.

Homologous recombination refers to a phenomenon where recombination of two DNA molecules occurs via the same nucleotide sequence within a cell and is a method frequently used for recombination of an organism having huge genomic DNA. A plasmid (called a targeting vector) is constructed by connecting foreign DNA in such a manner that the sequence of a target gene site is divided at the center. More specifically, the human G-CSF gene is isolated. A construct is prepared by sandwiching DNA of the gene between homologous sequences of the upstream part and the downstream part of a rodent's G-CSF receptor. The construct is inserted into a vector known in the technical field to prepare a targeting vector, which is then introduced into an immunodeficient rodent's ES cell (embryonic stem cell). Owing to homologous recombination, the rodent's G-CSF receptor gene DNA is exchanged with the same sequence part on the targeting vector. Since the foreign DNA sandwiched is integrated into a target gene, i.e., a G-CSF receptor gene, the gene loses function. In this manner, ES cells are established, injected in a rodent's embryo or blastocyst and transplanted into the uterus of a rodent having a pseudo-pregnancy with a chimera embryo to prepare a chimera mouse. The chimera mouse thus obtained is crossed with the aforementioned immunodeficient rodent to obtain F1 individuals having heterologous human G-CSF gene knock-ins at a rodent's G-CSF receptor locus. The individuals having heterologous knock-ins are crossed to successfully obtain an immunodeficient rodent having heterologous human G-CSF gene knock-ins at a rodent's G-CSF receptor locus.

At this time, as a vector for use in preparing a targeting vector, it is possible to use a vector that can be expressed in rodent's ES cells and can be used for transformation. For example, a plasmid derived from *Escherichia coli,* a retrovirus, a lentivirus, an adeno-associated virus and vaccinia virus, can be used.

A promoter may be linked to a site upstream of the human G-CSF gene. For example, a CMV promoter can be used.

A targeting vector can be introduced into an ES cell by a method known in the technical field such as an electroporation method, a calcium phosphate co-precipitation method, a lipofection method, a microinjection method and a particle gun method.

The vector may contain a selective marker gene. Examples of the marker gene include a hygromycin resistance gene, a neomycin resistance gene and a puromycin resistance gene. The marker gene may be removed after a homologous recombinant is selected and can be removed by use of the Cre-loxP system and Flp-frt system.

In place of ES cells, stem cells such as rodent's iPS cells can be used.

The genome editing is a method for modifying a target gene by use of a site specific nuclease. As a genome editing method, the following methods, which vary depending on the type of the nuclease to be used can be mentioned: ZFN (zinc finger nuclease) method (Urnov, Fyodor D. et al., Natur, Vol 435, 2 June 2005, pp.642-651), TALEN (Tale nuclease) method (Mahfouz, Magdy M et al., PNAS February 8, 2011, 108 (6), pp.2623-2628), methods using the CRISPR/Cas system such as CRISPR (Clustered Regularly Interspaced Short Palindromic Repeats)/Cas9 (Crispr Associated protein 9) (Jinek, Martin, et al., Science, Vol 337, 17 August 2012, pp.816-821) and CRISPR/Cas3. A method using a modified nuclease such as nickase modified Cas is also included in these methods. Of them, a method using the CRISPR/Cas9 system is preferred. In the CRSPR/Cas9 system, for example, a targeting vector containing guide RNA (crRNA, tracrRNA), which contains a complementary sequence for a target sequence of a rodent's G-CSF receptor gene, whose function is desired to destroy by cutting, a nuclease Cas9, and human G-CSF gene DNA to be knocked in as donor DNA, is injected into a fertilized egg of an immunodeficiency rodent gene. In the fertilized egg, the rodent's G-CSF receptor gene is deficient and, in place of the receptor gene, the human G-CSF gene is knocked in.

When a rodent's G-CSF receptor gene is destroyed by genome editing, a target sequence in the gene is selected and a guide RNA sequence may be designed so as to contain a complementary sequence for the sequence. The nucleotide length of the guide RNA is preferably 20 or more. The genome editing by CRSPR/Cas9 can be carried out by a commercially available CRISPR/Cas9 tool.

The strain of a knock-in mouse prepared having a human G-CSF gene knock-in at a G-CSF receptor locus of a NOG mouse is officially expressed as NOD.Cg-Prkdc^{scid}Il2rg^{tm1Sug}Csf3r^{tm1(CMV-CSF3)}/Jic and abbreviated as hG-CSF KI.

By transplanting human hematopoietic stem cells into the knock-in rodent prepared having a human G-CSF gene knock-in at a rodent's G-CSF receptor locus, a humanized rodent in which human hematopoietic stem cells are engrafted and human neutrophils circulate in the periphery, can be obtained.

It is preferable that the knock-in rodent is exposed to radiation before transplantation. At this time, it is preferable that radiation is applied at a dose of 0.5 to 1.5 Gy, preferably 0.8 to 1.3 Gy, and further preferably 1 Gy.

Human hematopoietic stem cells (CD34+ cells) are preferably transplanted via a vein, for example, a tail vein. The number of the human hematopoietic stem cells to be transplanted varies depending on the body weight of a rodent. In the case of a mouse, for example 10³ to 10⁶ human hematopoietic stem cells, preferably 10⁴ to 10⁵ human hematopoietic stem cells, and further preferably, 4 to 5 × 10⁴ human hematopoietic stem cells may be transplanted. A humanized rodent in which human neutrophils circulate in the periphery can be obtained 2 to 20 weeks, preferably 3 to 16 weeks, further preferably 4 to 12 weeks after transplantation.

### 2. Characteristics of humanized rodent having a human G-CSF gene knock-in at a rodent's G-CSF receptor locus and transplanted with human hematopoietic stem cells (HSC), in which human neutrophils circulate in the periphery

The humanized rodent having a human G-CSF gene knock-in at a rodent's G-CSF receptor locus prepared in step 1, and transplanted with human HSCs, in which human neutrophils circulate in the periphery, has the following characteristics.

The human G-CSF is present in blood and a rodent G-CSF receptor in cells is deficient. Because of this, the rodent's neutrophil count is low or zero.

Human neutrophils differentiate, increase and circulate in the peripheral blood. The ratio of human neutrophils 12 weeks after transplantation is 4 to 12%. The human neutrophils have an ability to produce reactive oxygen species.

Human neutrophils and monocytes differentiate, increase, and circulate in the peripheral blood. The ratio of human monocytes 12 weeks after transplantation is 10 to 40%.

Human cytokines derived from the bone marrow cells are present in the blood. Examples of the cytokines include IL-8, IL-6, IL-10, MCP-1, MIP1a, MIP-1b and IFNg.

In the knock-in rodent described herein transplanted with human hematopoietic stem cells and having the above characteristics, the human innate immune system can be reproduced.

Human neutrophils are increased by administering, for example, a TLR2 ligand, zymosan, with the result that the human cytokine concentration increases. Human neutrophils are increased by administering, for example, a bacterium such as *Escherichia coli,* with the result that emergency granulopoiesis, which occurs at the time of bacterial infection of a human, is reproduced. Human cytokine concentration is also increased by bacterial administration.

### 3. Use of humanized rodent having a human G-CSF gene knock-in at a rodent's G-CSF receptor locus and transplanted with human hematopoietic stem cells (HSC), in which human neutrophils circulate in the periphery

In the humanized rodent having a human G-CSF gene knock-in at a rodent's G-CSF receptor locus and transplanted with human hematopoietic stem cells (HSC), in which human neutrophils circulate in the periphery, the human innate immune system can be reproduced. Thus, the humanized rodent can be used in studies for human immunology.

If the rodent is infected with a bacterium, the rodent can be used in studies for the congenital defense mechanism of humans against bacterial infection.

Described herein is a rodent model for a human infectious disease prepared by infecting a humanized rodent having a human G-CSF gene knock-in at a rodent's G-CSF receptor locus and transplanted with human hematopoietic stem cells (HSC), in which human neutrophils are circulating in the periphery, with a bacterium or virus. Examples of the bacterium and virus include those pathogenic to humans, such as pathogenic *Escherichia coli,* enterohemorrhagic *Escherichia coli, Salmonella* spp., *Campylobacter jejunilcoli, Staphylococcus aureus, Vibrio parahaemolyticus, Clostridium botulinum, Bacillus cereus, Clostridium welch,* norovirus, hepatitis A virus and hepatitis E virus. A rodent model for a human infectious disease can be prepared by infecting the humanized rodent having a human G-CSF gene knock-in at a rodent's G-CSF receptor locus, in which human neutrophils are circulating in the periphery with these pathogens. The rodent model can be used in study for the defense mechanism of humans against an infectious disease and further for developing novel therapies and therapeutic drugs.

### 4. hG-CSF KI, FcR KO rodent

Described herein is a rodent having a human G-CSF gene knock-in at a rodent's G-CSF receptor locus and further deficient in Fcer1g and Fcgr2b, obtained by crossing the aforementioned rodent having a human G-CSF gene knock-in with a rodent deficient in Fcer1g and Fcgr2b, which are constituent molecules of a receptor that binds to the Fc region of an antibody.

Human neutrophils differentiate, increase and circulate in the peripheral blood by transplanting human hematopoietic stem cells (CD34+ cells) to the rodent. Human hematopoietic stem cells (CD34+ cells) may be transplanted in accordance with the method mentioned above.

Examples of the rodent include a rodent having a declined immune function or no immune function and inactivated immune response to humans. Examples of the immunodeficient rodent are the same as mentioned above. Of them, a mouse, particularly a NOG mouse, is preferable.

Examples of the mouse include a hG-CSF KI/FcR KO mouse, which is obtained by crossing a hG-CSF KI mouse with an FcR KO mouse (Inoue Y et al. Journal of immunology 2007; 179: 764-774.) deficient in Fcerlg and Fcgr2b, which are constituent molecules of a receptor that binds to the Fc region of an antibody. Note that, the hG-CSF KI/FcR KO mouse can be prepared by deleting an Fcerlg gene and Fcgr2b gene by genome editing.

The mouse has a human G-CSF gene knock-in at a G-CSF receptor locus of a NOG mouse and is deficient in Fcerlg and Fcgr2b.

Human neutrophils differentiate, increase and circulate in the peripheral blood by transplanting human hematopoietic stem cells (CD34+ cells) into the mouse. The ratio of the human neutrophils 4 weeks after transplantation is 15 to 20%. The human neutrophils have an ability to produce reactive oxygen species.

Described herein is a rodent model for a human infectious disease obtained by infecting the above rodent with a bacterium or virus. Examples of the bacterium or virus are the same as mentioned above.

### 5. hIL-3/hGM-CSF Tg, FcR KO rodent and hIL-3/hGM-CSF/hIL-5 Tg, FcR KO rodent

Described herein is a rodent having human IL-3 and GM-CSF genes inserted in the genome and deficient in Fcerlg and Fcgr2b, obtained by crossing a rodent having human IL-3 and GM-CSF genes inserted in the genome with a rodent deficient in Fcer1g and Fcgr2b, which are constituent molecules of a receptor that binds to the Fc region of an antibody.

Human eosinophils differentiate, increase and circulate in the peripheral blood by transplanting human hematopoietic stem cells (CD34+ cells) into the rodent.

Examples of the immunodeficient rodent are the same as mentioned above. Of them, a mouse, particularly, a NOG mouse, is preferable.

In the case of a mouse, a hIL-3/hGM-CSF Tg, FcR KO mouse is mentioned, which is obtained by crossing a hIL-3/hGM-CSF Tg mouse (Ito R et al. Journal of immunology 2013; 191: 2890-2899.) with an FcR KO mouse. Note that, a hIL-3/hGM-CSF Tg, FcR KO mouse can be prepared by deleing an Fcerlg gene and Fcgr2b gene by genome editing.

Human eosinophils differentiate, increase, and circulate in the peripheral blood by transplanting human hematopoietic stem cells (CD34+ cells) into the mouse. The ratio of human eosinophils 4 weeks after transplantation is 10 to 15%.

Described herein is a rodent having human IL-3, GM-CSF and IL-5 genes inserted in the genome and deficient in Fcer1g and Fcgr2b, obtained by crossing a rodent having human IL-3, GM-CSF and IL-5 gene inserts in the genome with a rodent deficient in Fcerlg and Fcgr2b, which are constituent molecules of a receptor that binds to the Fc region of an antibody.

Human eosinophils and human basophils differentiate, increase, and circulate in the peripheral blood by transplanting human hematopoietic stem cells (CD34+ cells) into the rodent.

Examples of the immunodeficient rodent are the same as mentioned above. Of them, a mouse, particularly, a NOG mouse, is preferable. The rodent having human IL-3, GM-CSF and IL-5 gene inserts in the genome secretes human IL-3, GM-CSF and IL-5 cytokines. After human hematopoietic stem cells are transplanted into the rodent, human cells such as eosinophils, basophils and mast cells can differentiate.

In the case of a mouse, a hIL-3/hGM-CSF/hIL-5 Tg, FcR KO mouse is mentioned, which is obtained by crossing a hIL-3/hGM-CSF/hIL-5 Tg mouse (Ito R et al., JCI Insight. 2018 Nov 2; 3 (21)) with an FcR KO mouse. Note that, a hIL-3/hGM-CSF/hIL-5 Tg, FcR KO mouse can be prepared by deleting an Fcerlg gene and Fcgr2b gene by genome editing.

Human eosinophils differentiate, increase, and circulate in the peripheral blood by transplanting human hematopoietic stem cells (CD34+ cells) into the mouse. The ratio of human eosinophils 4 weeks after transplantation is 20 to 30%.

Human basophils differentiate, increase, and circulate in the peripheral blood by transplanting human hematopoietic stem cells (CD34+ cells) into the mouse. The ratio of human basophils 4 weeks after transplantation is 4 to 8%.

Described herein is a rodent model for a human infectious disease obtained by infecting the above rodent with a bacterium or virus. Examples of the bacterium and virus are the same as mentioned above.

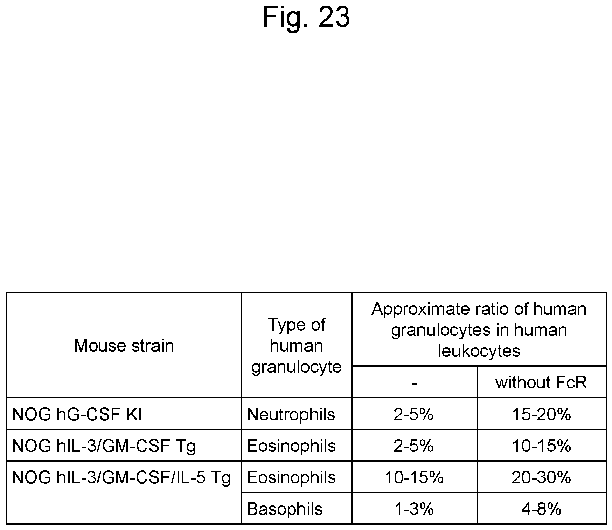

The relationships between mouse strains and the ratios of human granulocytes in human leukocytes are shown in Figure 23.

### Examples

The following examples are not necessarily according to the invention and are present for illustration purposes only.

### [Example 1] Human G-CSF knock-in mouse in which human neutrophils differentiate

### 1. Preparation of KI targeting vector

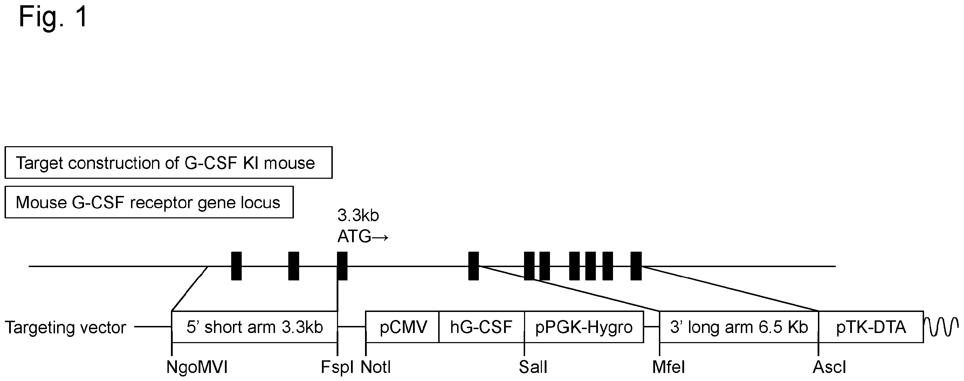

A KI targeting vector was prepared by placing a human G-CSF gene directly downstream of a systemic expression promoter, i.e., cytomegalovirus (CMV) promoter, and sandwiching it between homologous sequences, that is, a sequence (3.3 Kb) upstream of a mouse G-CSF receptor region and a sequence (6.5 Kb) downstream thereof. The structure of the targeting vector is shown in Figure 1.

### 2. Preparation of human G-CSF knock-in mouse

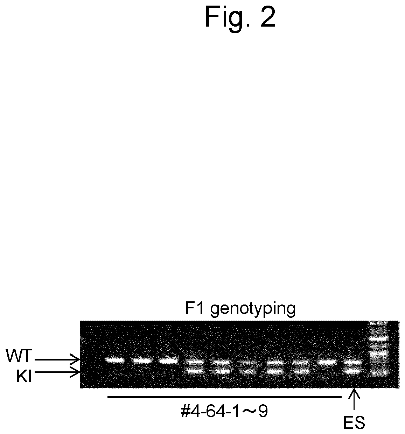

The targeting vector prepared as mentioned above was introduced in NOG-mouse ES cells by electroporation to establish homologous recombination ES cells. Thereafter, chimera mice were prepared and crossed with NOG mice to prepare F1 mice. A G-CSF receptor gene was analyzed by a PCR method. The results of the gene analysis are shown in Figure 2. The mouse strain established is officially expressed as NOD.Cg-Prkdc^{scid}Il2rg^{tm1Sug}Csf3r^{tm1(CMV-CSF3)}/Jic and abbreviated as hG-CSF KI.

### 3. Evaluation of hG-CSF KI mouse

### (1) Confirmation of expression of human G-CSF

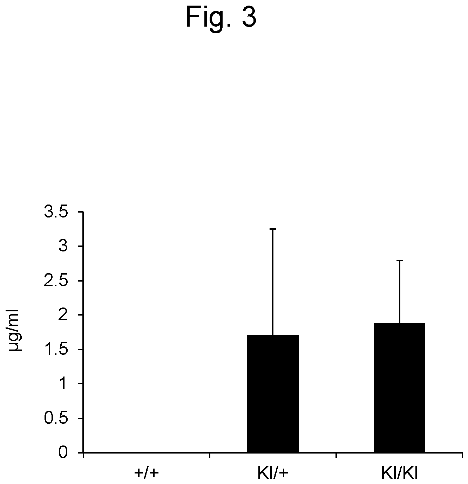

Blood was taken from 6 to 8 weeks-old hG-CSF KI mice (hetero, homo) and NOG mice by use of heparin and plasma was separated by a centrifugal operation. The level of human G-CSF cytokine was measured by a Human G-CSF Quantikine ELISA Kit (R&D systems). The results are shown in Figure 3. The levels of the human G-CSF cytokine in KI/+ hetero mice and KI/KI homo mice were as high as 1.69 µg/ml and 1.87 µg/ml in concentration, respectively.

### (2) Expression of mouse G-CSF receptor

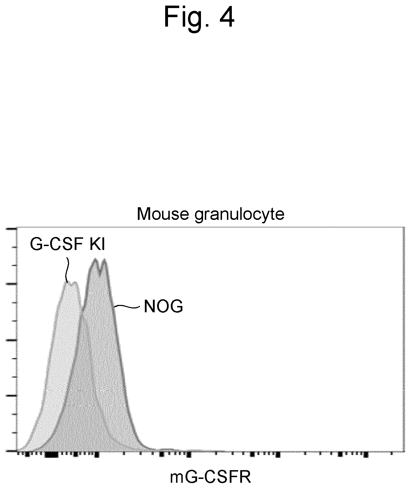

Blood was taken from 6 to 8 weeks-old hG-CSF KI mice and NOG mice by use of heparin. White blood cells were separated by lysing red blood cells and stained with an anti-mouse G-CSF receptor antibody. Thereafter, expression thereof was measured by a flow cytometer. The results are shown in Figure 4. G-CSFR was not expressed in the granulocytes of KI/KI homo mice. From this, it was demonstrated that a loss in expression is due to gene disruption.

### (3) Confirmation of mouse neutrophil count

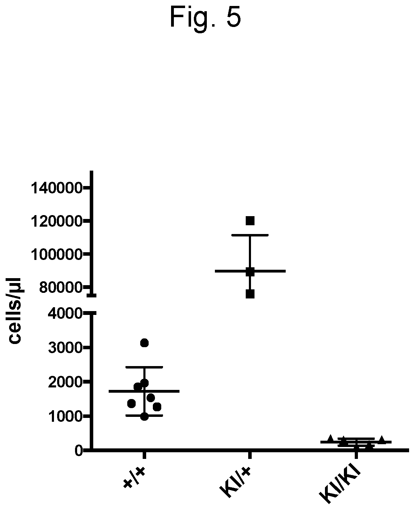

Blood was taken from 6 to 8 weeks-old hG-CSF KI mice (hetero, homo) and NOG mice by use of heparin. White blood cells were separated by lysing red blood cells and stained with an anti-mouse Gr1 antibody. Thereafter, mouse neutrophil count was analyzed by flow cytometry. The results are shown in Figure 5. The mouse neutrophil count significantly increased in KI/+ and extremely low in KI/KI. From this, it was demonstrated that a decrease of mouse neutrophil count is due to defect of a mouse G-CSF receptor in the KI/KI mouse.

### 4. Humanization of human G-CSF knock-in mouse

### (1) Radiation sensitivity test

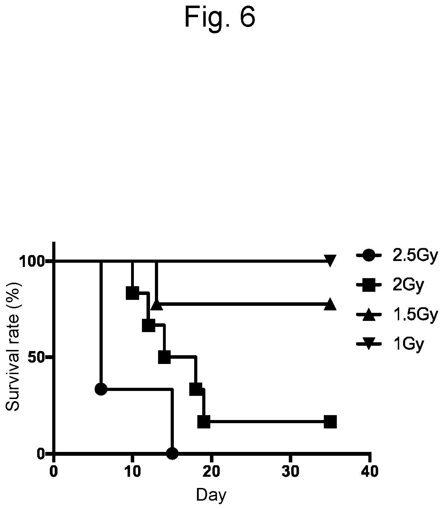

Radiation was applied to 6 to 8 weeks-old hG-CSF KI mice at a dose of 1, 1.5, 2, and 2.5 Gy. The mice were observed for a month or more and the death rate was determined. The results of the radiation sensitivity test are shown in Figure 6. The hG-CSF KI mice had high radiation sensitivity compared to the NOG mice. About 90% of the mice died at a dose of 2 Gy and about 20% of mice died even at a dose of 1.5 Gy. From this, a dose of 1 Gy was employed as an optimal irradiation condition.

### (2) Human neutrophils confirmed

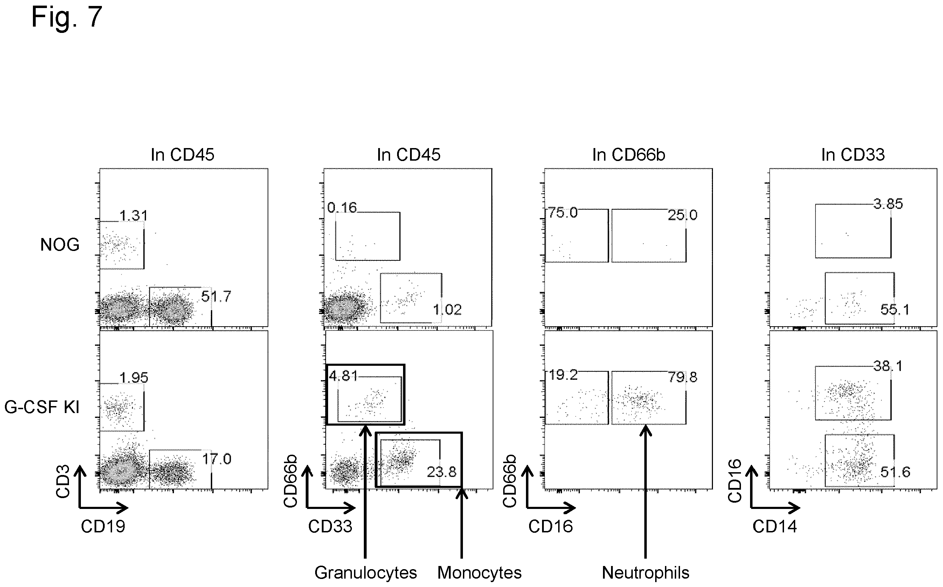

Radiation was applied to 6 to 8 weeks-old hG-CSF KI mice at a dose of 1 Gy. At the following day, 4 to 5 × 10⁴ human CD34+ cells (HSC: hematopoietic stem cells) were transplanted via the tail vein. Twelve weeks after transplantation, the peripheral blood was taken and the ratios of human leukocyte fractions, i.e., human neutrophils and monocytes, were analyzed by a flow cytometer. The results are shown in Figure 7. It was found that the ratios of CD66b+ granulocytes and CD33+ monocytes in human leukocytes (CD45) increased in the G-CSF KI mice, and that most of the granulocytes consisted of CD16+ neutrophils. The fractions of neutrophils and monocytes in the NOG mice were extremely low.

### (3) Confirmation of chimera rate of human cells

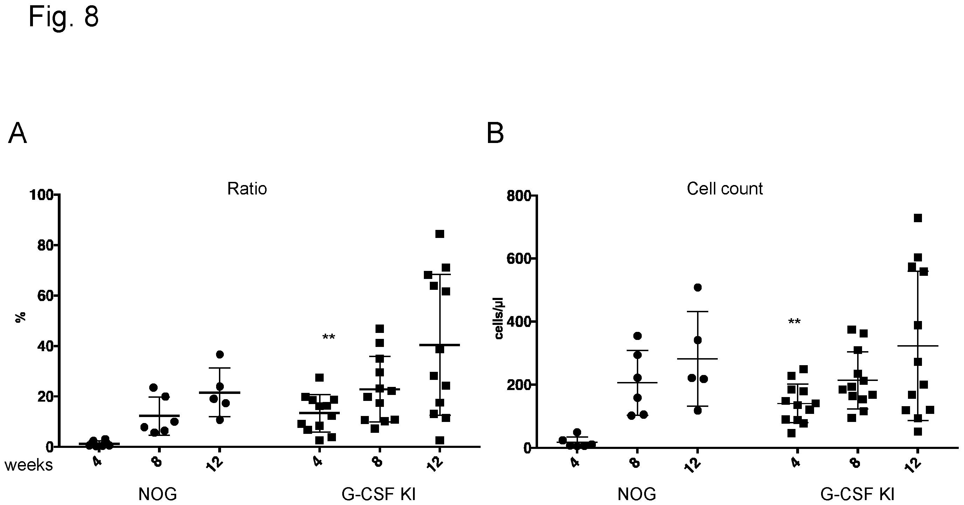

The ratio and count of human CD45+ cells (human leukocytes) shown in Figure 7 were analyzed over time, at 4, 8 and 12 weeks after transplantation. The results are shown in Figure 8. The ratio and count of the cells both were high in KI mice 4 weeks after humanization but no significant difference was observed thereafter.

### (4) Ratio of human neutrophils

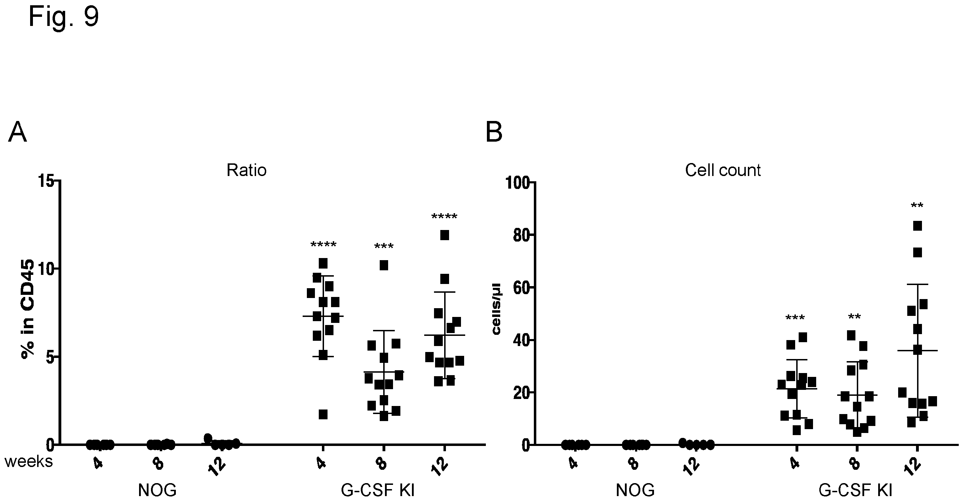

The ratio and count of the human CD66b+CD16+ cells (human neutrophils) shown in Figure 7 were analyzed over time, at 4, 8 and 12 weeks after transplantation. The results are shown in Figure 9. The ratio and count both were significantly high in the KI mice during the whole period after humanization.

### (5) Ratio of human monocytes

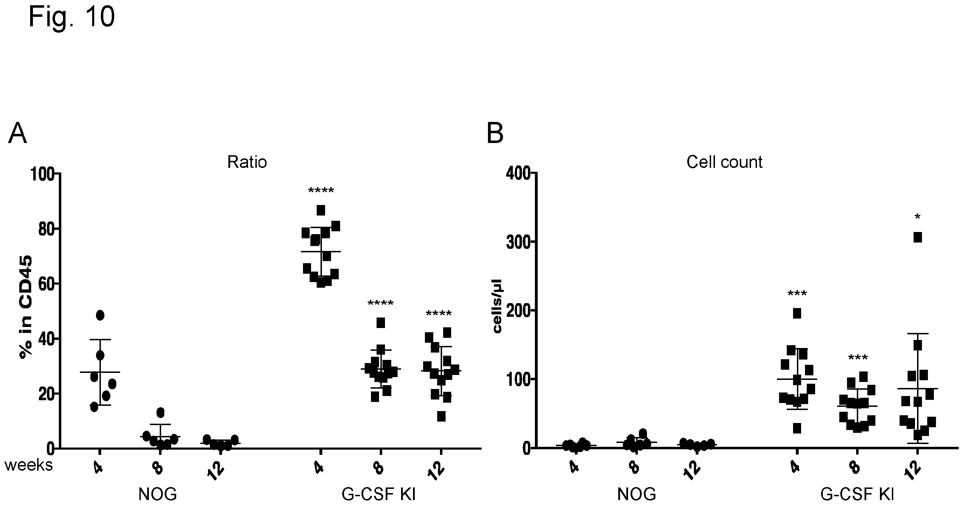

The ratio and count of the human CD33+CD14+ cells (human monocytes) shown in Figure 7 were analyzed over time, at 4, 8 and 12 weeks after transplantation. The results are shown in Figure 10. The ratio and count both were significantly high in KI mice during the whole period after humanization.

### (6) Confirmation of morphology of human neutrophils

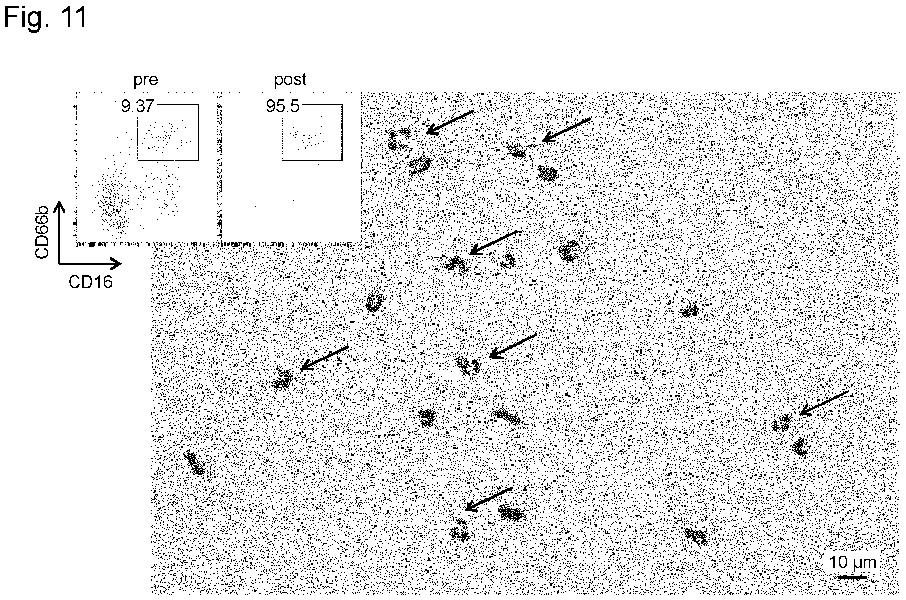

Blood was taken from hG-CSF KI mice 12 weeks after humanization and stained with a human neutrophil marker. Neutrophil fractions were separated by a cell sorter and subjected to Giemsa staining to observe morphology of the cells. The results are shown in Figure 11. Many cells having neutrophil-like morphology having multilobed nuclei were observed.

### (7) Measurement of reactive oxygen species production ability of human neutrophils

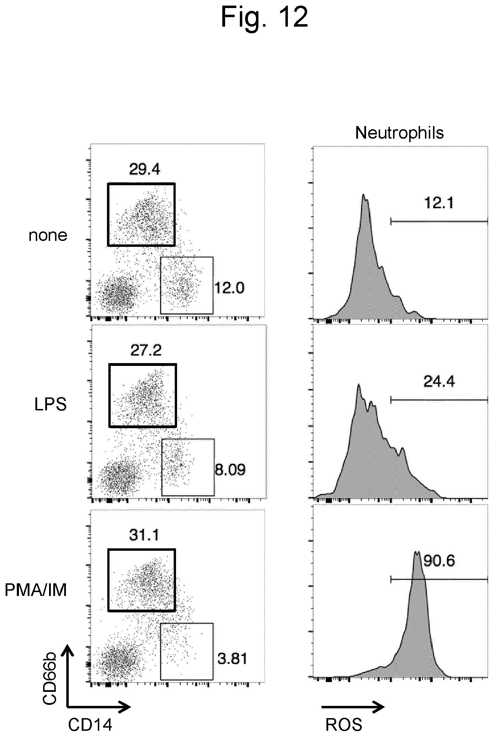

Blood was taken from hG-CSF KI mice 12 weeks after humanization. After white blood cells were isolated and stimulated *in vitro* with LPS (1 µg/ml) or PMA (20 ng/ml), ROS production ability was analyzed by flow cytometry. The results are shown in Figure 12. When neutrophils were stimulated with LPS, 20 to 30% of them were ROS positive. When neutrophils were stimulated with PMA, 80 to 90% of them were ROS positive. It was found that the human neutrophils have an ROS producing ability.

### (8) Production of myeloid-derived human cytokine

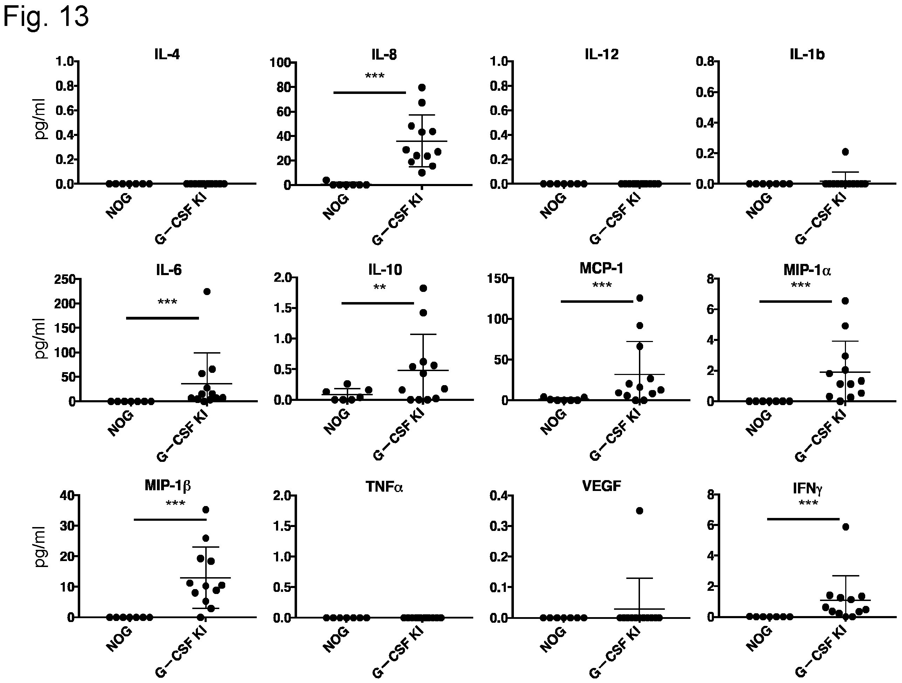

Blood was taken from the hG-CSF KI mice and NOG mice 16 to 20 weeks after humanization. After plasma was separated, levels of various human cytokines were determined by Cytokine Beads Array (CBA: BD Bioscience). The results are shown in Figure 13. Productions of IL-8, IL-6, IL-10, MCP-1, MIP1a, MIP-1b and IFNg significantly increased in G-CSF KI mice.

### (9) Ratio of human neutrophils after administration of a TLR2 ligand, zymosan

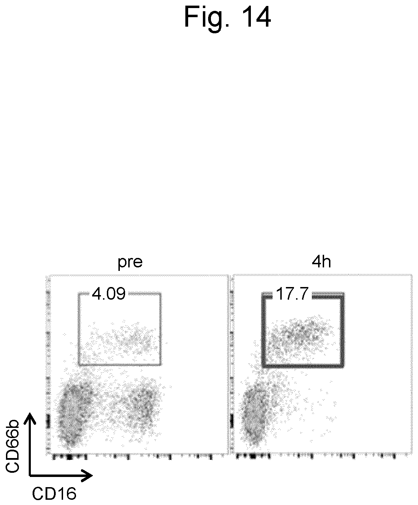

To the hG-CSF KI mice and NOG mice 12 weeks after humanization, 1 mg/200 µL of a zymosan solution was intraperitoneally administered. Four hours later, blood was taken and subjected to flow cytometric analysis for human neutrophils. The results are shown in Figure 14. The ratio of human neutrophils before administration of zymosan was about 4% and increased up to about 20% 4 hours after administration.

### (10) Ratio of human cells after administration of zymosan

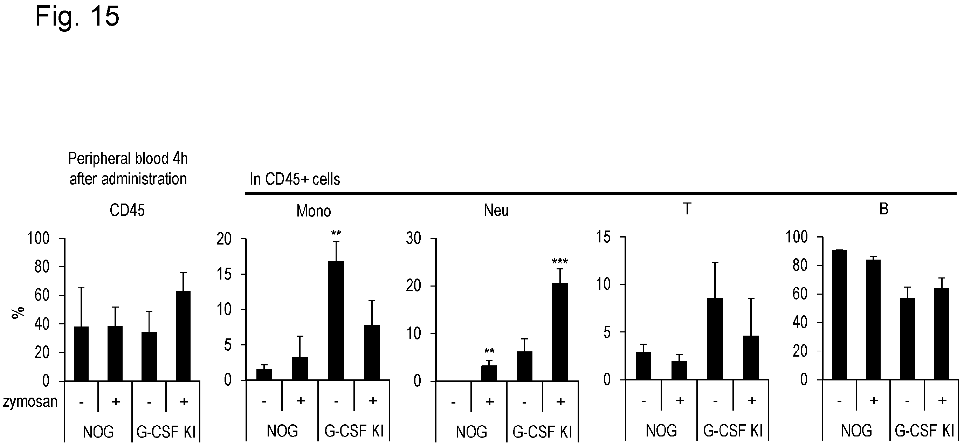

The flow-cytometry analysis results of human CD45-positive white blood cells, monocytes, neutrophils, T cells and B cells in the experiment shown in Figure 14 are collectively shown in Figure 15. In the hG-CSF KI mice, the ratio of human monocytes decreased and the ratio of human neutrophils increased after administration of zymosan. Also in the NOG mice, the human neutrophils increased but the ratio was as low as about 4%.

### (11) Measurement of human cytokines in serum

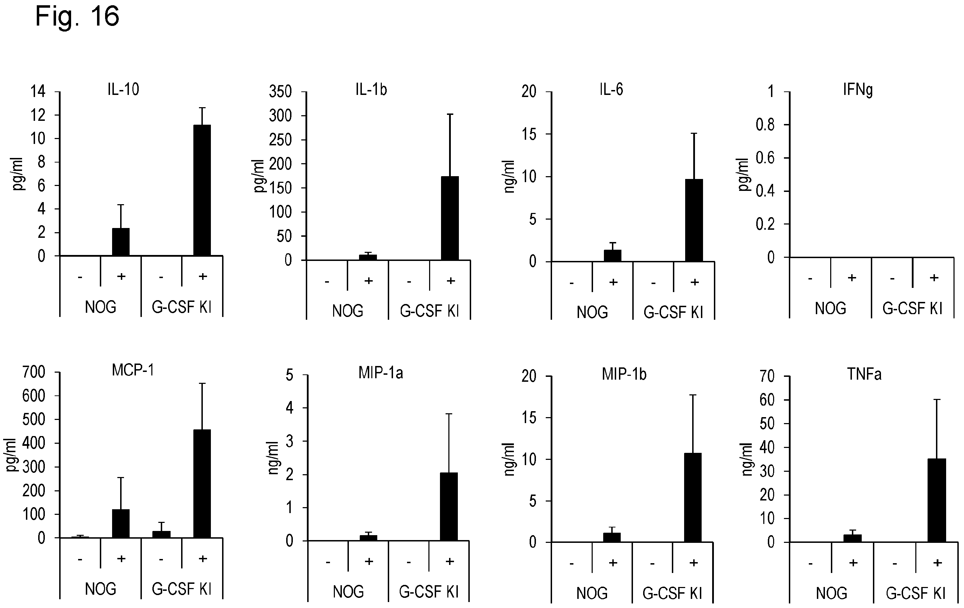

In the experiment shown in Figure 14, plasma was taken and levels of various human cytokines in the plasma were determined by Cytokine Beads Array (CBA: BD Bioscience). The results are shown in Figure 16. In hG-CSF KI mice, productions of human IL-10, IL-1b, IL-6, MCP-1, MIP1a, MIP-1b and TNFa remarkably increased after administration of zymosan, demonstrating that innate immune response via human neutrophils, monocytes/macrophages increases compared to those in NOG mice.

### (12) Ratio of human cells after administration of Escherichia coli

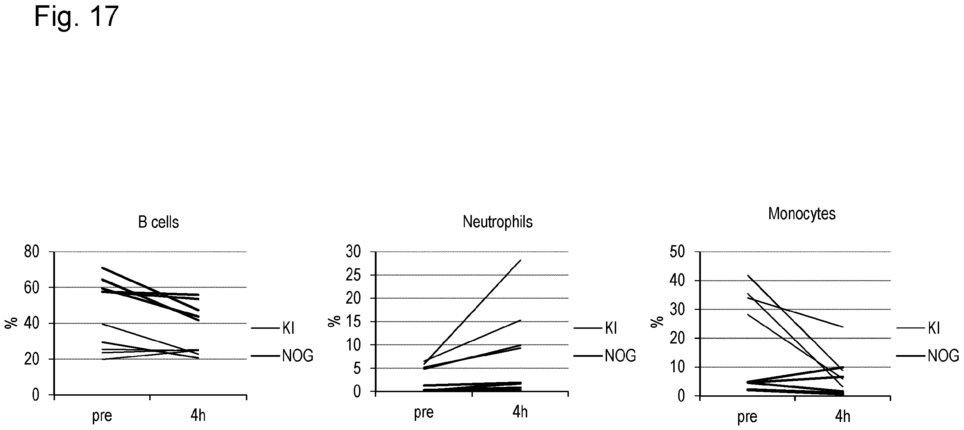

An *Escherichia coli* solution (4 × 10⁸ cfu) was intraperitoneally administered to the hG-CSF KI mice and NOG mice 14 weeks after humanization. Four hours later, blood was taken and human neutrophils, monocytes and B cells were analyzed by flow cytometry. The results are shown in Figure 17. The human neutrophils significantly increased after administration of *Escherichia coli.* Emergency granulopoiesis occurring at the time of bacterial infection of a human was reproduced.

### (13) Measurement of cytokines after administration of Escherichia coli

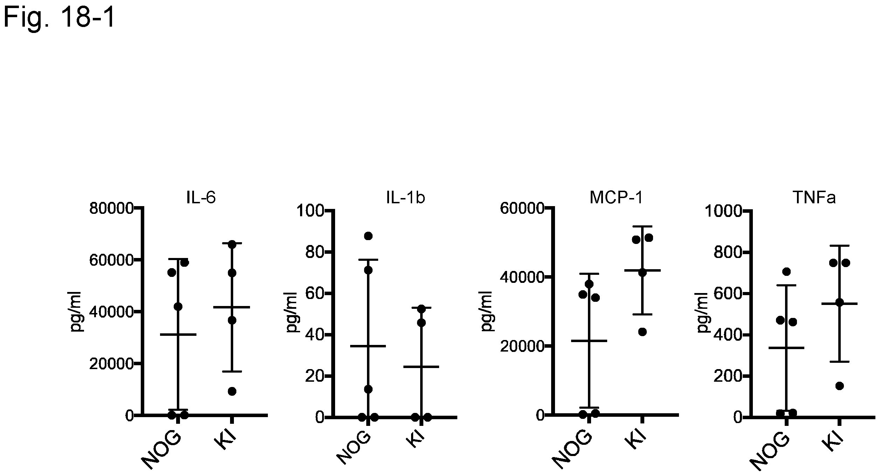

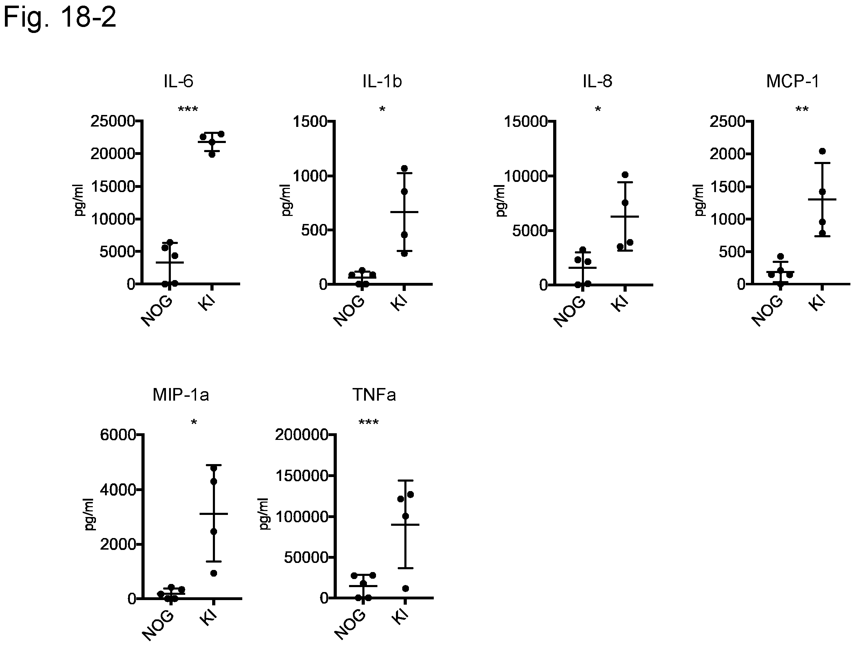

In the experiment shown in Figure 17, plasma was taken, levels of various mouse and human cytokines in the plasma were determined by Cytokine Beads Array (CBA: BD Bioscience). The quantitative results of mouse cytokines are shown in Figure 18-1 and the quantitative results of human cytokines are shown in Figure 18-2. No difference was observed between the NOG mice and the hG-CSF KI mice with respect to the mouse cytokines. Productions of human cytokines, IL-6, IL-1b, IL-8, MCP-1, MIP1a and TNFa significantly increased in G-CSF KI mice. From these, the G-CSF KI mice are considered as humanized mice in which emergency granulopoiesis after bacterial infection and innate immune response accompanied with increased human cytokines can be analyzed and useful as a model for developing a therapeutic drug for sepsis induced by bacterial infection.

### [Example 2] Increase of human neutrophils by crossing with FcR KO mice

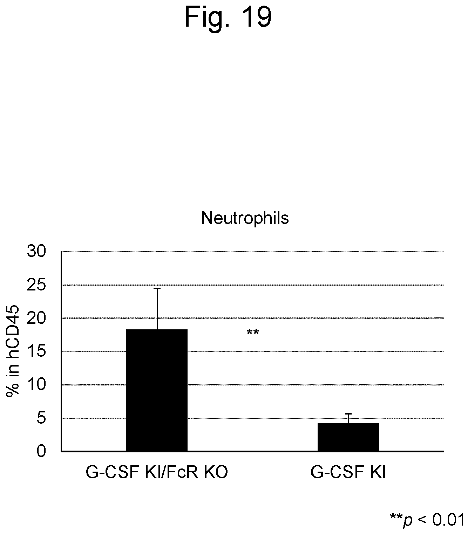

FcR KO mice deficient in Fcer1g and Fcgr2b, which are constituent molecules of a receptor that binds to the Fc region of an antibody, have been already established (Inoue Y et al., Journal of immunology 2007; 179: 764-774). The FcR KO mice were crossed with hG-CSF KI mice to newly establish hG-CSF KI, FcR KO mice. When human hematopoietic stem cells were transplanted into hG-CSF KI, FcR KO mice, it was found that the ratio of human neutrophils in human leukocytes of mice peripheral blood is about 2 to 5% in the hG-CSF KI mice, whereas the ratio is 15 to 20% (4 times higher) in the hG-CSF KI, FcR KO mice (Figure 19). Neutrophils differentiated from hematopoietic stem cells in the bone marrow in a G-CSF dependent manner, more differentiated in response to stimulation such as infection and quickly migrate to the site stimulated to successfully remove bacteria.

### [Example 3] Increase of human eosinophils by crossing with FcR KO mice

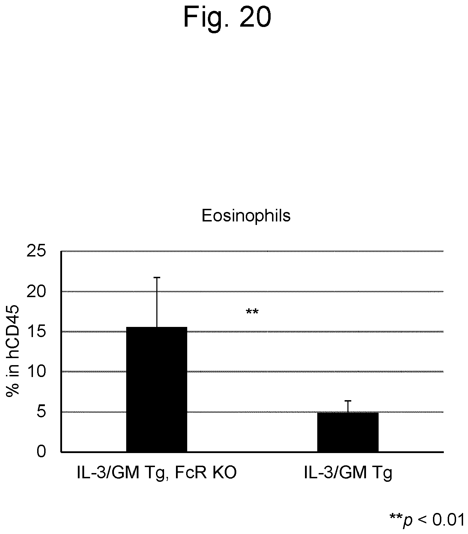

hIL-3/hGM-CSF Tg mice that the present inventors already reported (Ito R et al. Journal of immunology 2013; 191: 2890-2899.) were crossed with the FcR KO mice to newly establish hIL-3/hGM-CSF Tg, FcR KO mice. When human hematopoietic stem cells were transplanted to hIL-3/hGM-CSF Tg, FcR KO mice, it was found that the ratio of human eosinophils in peripheral blood is about 2 to 5% in hIL-3/hGM-CSF Tg mice, whereas the ratio is 10 to 15% (three times higher) in hIL-3/GM-CSF Tg, FcR KO mice (Figure 20).

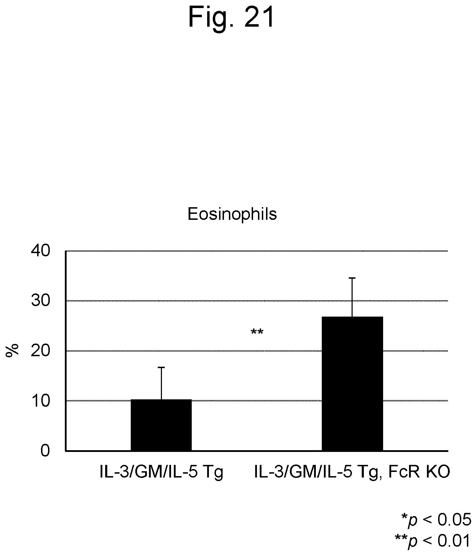

Similarly, hIL-3/hGM-CSF/hIL-5 Tg mice (Ito R et al., JCI Insight. 2018 Nov 2; 3 (21)) were crossed with FcR KO mice to newly establish hIL-3/hGM-CSF/hIL-5 Tg, FcR KO mice. When human hematopoietic stem cells were transplanted into hIL-3/hGM-CSF/hIL-5 Tg, FcR KO mice, it was found that the ratio of human eosinophils in human leukocytes of mouse peripheral blood is about 10 to 15% in the hIL-3/hGM-CSF Tg mice, whereas the ratio in the hIL-3/hGM-CSF/hIL-5/FcR KO mice is 20 to 30% (about twice as high) (Figure 21). Eosinophils differentiate from hematopoietic stem cells in the bone marrow and are increased by a cytokine such as IL-5. Highly invasion in tissue was observed in response to inflammation via an allergen at a local site such as a lung tissue.

### [Example 4] Increase of human basophils by crossing with FcR KO mice

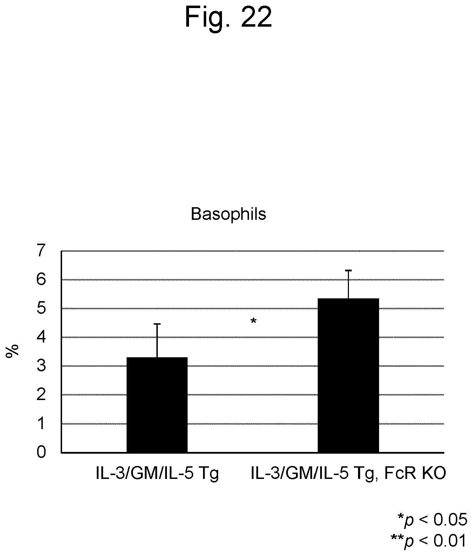

When human hematopoietic stem cells were transplanted to hIL-3/hGM-CSF/hIL-5 Tg mice and hIL-3/hGM-CSF/hIL-5 Tg, FcR KO mice and the ratio of human basophils in human leukocytes of mouse peripheral blood was determined, it was found that the ratio in hIL-3/hGM-CSF/hIL-5 Tg mice is about 1 to 3%, whereas the ratio is 4 to 8% (about twice as high) in hIL-3/hGM-CSF/hIL-5 Tg, FcR KO mice (Figure 22). Basophils differentiate from hematopoietic stem cells in the bone marrow and are increased by a cytokine such as IL-3. Basophils migrate to a local site during allergic inflammation and exacerbate inflammation.

### Industrial Applicability

A human G-CSF knock-in rodent in which human neutrophils differentiate can be used in studies for the human immune system and as a human infection model mouse. Further, the human G-CSF knock-in rodent, if it is crossed with an FcR KO mouse, can more efficiently induce granulocytes, and thus, can be used as a highly sensitive infectious disease model and allergy model.

## Claims

1. A rodent having a human G-CSF gene knock-in at a G-CSF receptor locus thereof, which is an immunodeficient rodent deficient in G-CSF receptor function by knock-in of the human G-CSF gene at the G-CSF receptor locus thereof, wherein the human G-CSF is expressed and the rodent G-CSF receptor is not expressed, and further deficient in Fcer1g and Fcgr2b, which are constituent molecules of a receptor that binds to an Fc region of an antibody, obtained by further deleting the Fcer1g and Fcgr2b in the rodent.

2. The rodent of claim 1 wherein the rodent is a humanized rodent having human neutrophils circulating in a periphery, obtained by transplanting a human hematopoietic stem cell into the rodent according to claim 1.

3. A rodent having human IL-3 and human GM-CSF genes inserted and further deficient in Fcer1g and Fcgr2b, which are constituent molecules of a receptor that binds to an Fc region of an antibody, obtained by further deleting the Fcer1g and Fcgr2b in a rodent having human IL-3 and human GM-CSF genes inserted.

4. The rodent of claim 3 wherein the rodent is a humanized rodent having human eosinophils circulating in a periphery, obtained by transplanting a human hematopoietic stem cell into the rodent according to claim 3.

5. The rodent of claim 3 wherein the human IL-5 gene is inserted, obtained by deleting the Fcer1g and Fcgr2b in a rodent having human IL-3, GM-CSF and IL-5 genes inserted.

6. The rodent of claim 5 wherein the rodent is a humanized rodent having human eosinophils and basophils circulating in a periphery, obtained by transplanting a human hematopoietic stem cell into the rodent according to claim 5.

7. A rodent model for a human pathogen infectious disease, obtained by infecting the humanized rodent of claims 2, 4 or 6 with a bacterium or virus.

## Patentansprüche

1. Nager, aufweisend einen Knock-in des humanen G-CSF-Gens an einem G-CSF-Rezeptorlocus davon, wobei es sich um einen immundefizienten Nager handelt, der hinsichtlich der G-CSF-Rezeptorfunktion durch den Knock-in des humanen G-CSF-Gens an seinem G-CSF-Rezeptorlocus defizient ist, wobei das Human-G-CSF exprimiert wird und der Nager-G-CSF-Rezeptor nicht exprimiert wird, und der ferner hinsichtlich Fcerlg und Fcgr2b defizient ist, die Bestandteilmoleküle eines Rezeptors sind, der an eine Fc-Region eines Antikörpers bindet, erhalten dadurch, dass weiterhin das Fcerlg und Fcgr2b in dem Nager deletiert wurden.

2. Nager nach Anspruch 1, wobei der Nager ein humanisierter Nager, aufweisend in der Peripherie zirkulierende humane Neutrophile, ist, der durch Transplantieren einer humanen hämatopoetischen Stammzelle in den Nager gemäß Anspruch 1 erhalten wird.

3. Nager, bei dem das humane IL-3- und das humane GM-CSF-Gen insertiert sind und der ferner hinsichtlich Fcerlg und Fcgr2b defizient ist, die Bestandteilmoleküle eines Rezeptors sind, der an eine Fc-Region eines Antikörpers bindet, erhalten dadurch, dass weiterhin das Fcerlg und Fcgr2b in einem Nager deletiert wurden, bei dem das humane IL-3- und das humane GM-CSF-Gen insertiert sind.

4. Nager nach Anspruch 3, wobei der Nager ein humanisierter Nager, aufweisend in der Peripherie zirkulierende humane Eosinophile, ist, der durch Transplantieren einer humanen hämatopoetischen Stammzelle in den Nager gemäß Anspruch 3 erhalten wird.

5. Nager nach Anspruch 3, bei dem das humane IL-5-Gen insertiert ist, erhalten dadurch, dass das Fcerlg und Fcgr2b in einem Nager deletiert wurden, bei dem humane IL-3-, GM-CSF- und IL-5-Gene insertiert sind.

6. Nager nach Anspruch 5, wobei der Nager ein humanisierter Nager, aufweisend in der Peripherie zirkulierende humane Eosinophile und Basophile, ist, der durch Transplantieren einer humanen hämatopoetischen Stammzelle in den Nager gemäß Anspruch 5 erhalten wird.

7. Nagermodell für eine humanpathogene Infektionskrankheit, das durch Infizieren des humanisierten Nagers nach Anspruch 2, 4 oder 6 mit einem Bakterium oder Virus erhalten wird.

## Revendications

1. Rongeur ayant un gène knock-in de G-CSF humain au niveau d'un locus de récepteur de G-CSF correspondant, qui est un rongeur immunodéficient déficient en fonction de récepteur de G-CSF par knock-in du gène de G-CSF humain au niveau du locus du récepteur de G-CSF correspondant, le G-CSF humain étant exprimé et le récepteur de G-CSF de rongeur n'étant pas exprimé, et en outre déficient en Fcerlg et Fcgr2b, qui sont des molécules constituantes d'un récepteur qui se lie à une région Fc d'un anticorps, obtenu par délétion supplémentaire des Fcerlg et Fcgr2b chez le rongeur.

2. Rongeur selon la revendication 1, le rongeur étant un rongeur humanisé ayant des neutrophiles humains circulant dans une périphérie, obtenu par transplantation d'une cellule souche hématopoïétique humaine dans le rongeur selon la revendication 1.

3. Rongeur ayant des gènes d'IL-3 humaine et de GM-CSF humain insérés et en outre déficient en Fcerlg et Fcgr2b, qui sont des molécules constituantes d'un récepteur qui se lie à une région Fc d'un anticorps, obtenu par délétion supplémentaire des Fcerlg et Fcgr2b chez un rongeur ayant des gènes d'IL-3 humaine et de GM-CSF humain insérés.

4. Rongeur selon la revendication 3, le rongeur étant un rongeur humanisé ayant des éosinophiles humains circulant dans une périphérie, obtenu par transplantation d'une cellule souche hématopoïétique humaine dans le rongeur selon la revendication 3.

5. Rongeur selon la revendication 3, le gène d'IL-5 humain étant inséré, obtenu par délétion des Fcerlg et Fcgr2b chez un rongeur ayant des gènes d'IL-3, GM-CSF et IL-5 humains insérés.

6. Rongeur selon la revendication 5, le rongeur étant un rongeur humanisé ayant des éosinophiles et basophiles humains circulant dans une périphérie, obtenu par transplantation d'une cellule souche hématopoïétique humaine dans le rongeur selon la revendication 5.

7. Modèle de rongeur pour une maladie infectieuse pathogène humaine, obtenu par infection du rongeur humanisé selon les revendications 2, 4 ou 6 avec une bactérie ou un virus.
